# EUROPEAN PATENT SPECIFICATION

(11) **EP 1 804 814 B1**
(45) Date of publication and mention of the grant of the patent: **11.12.2013**
(21) Application number: 05798295.1
(22) Date of filing: 21.09.2005
(51) Int. Cl.: A61K 33/06, A61K 33/08, A61K 33/42, A61K 47/36, A61K 47/38, A61K 47/26, A61K 47/02, A61K 31/726, A61K 31/715, A61K 31/716, A61K 31/717, A61K 31/718, A61K 31/739, A61K 31/7004, A61K 31/7016, A61K 31/702, A61F 2/28

(54) **MULTI-PURPOSE BIO-MATERIAL COMPOSITION**
MEHRZWECK-ZUSAMMENSETZUNG AUS BIOLOGISCHEM MATERIAL
COMPOSITION DE BIOMATERIAU POLYVALENTE

(30) Priority: 21.09.2004 US 611840 P
(43) Date of publication of application: 11.07.2007
(73) Proprietor: Lally, Thomas Joseph, Oak Brook, IL 60523 (US)
(72) Inventor: Lally, Thomas Joseph, Oak Brook, IL 60523 (US)
(74) Representative: Mallalieu, Catherine Louise
(86) International application number: PCT/US2005/034035
(87) International publication number: WO 2006/034420

(56) References cited:
- WO-A1-01/41822
- DE-A1- 2 756 256
- US-A1- 2003 199 615
- US-A1- 2005 031 704
- US-B1- 6 533 821
- US-B1- 6 733 582

## Description

### Technical Field

The present invention relates to a bio-material composition. More specifically the invention relates to a multi-purpose, phosphate-based bio-material useful as a bone filler, bio-adhesive, bone cement and bone graft. The present invention is particularly useful as a bio-adhesive for bone, ligament, and other soft tissue and has surprising osteoproliferative effects. The invented binder composition has a variety of other uses.

### Background Art

Increasing numbers of sports and age related injuries like broken bones, wom out joints, and tom ligaments have heightened the demand for bio-materials capable of treating orthopedic injuries. In response, companies have developed bone cements to attach various objects to bone, and bone fillers capable of treating bone fractures and other bone defects. However, existing absorbable bio-materials are inadequate at supplementing the reattachment of soft tissues like ligaments to bone and stimulating new bone formation.

Most existing bio-materials are made of calcium phosphates or relatively inert hardening polymers like polymethylmethcrylate ("PMMA").

U.S. Patent No. 5,968,999 issued to Ramp et al, describes a PMMA based bone cement composition useful for orthopedic procedures. Unfortunately, PMMA-based bio-materials release considerable amounts of heat to the surrounding bone during the curing process causing cell death. The resulting materials shrink during setting and have poor resistance to fracture. PMMA biomaterials also possess slow rates of bio-absorption and poor bio-compatibility due to the release of a toxic monomer into the blood stream. There is little evidence that PMMA based materials promote any significant new bone formation.

A number of calcium phosphate based compositions have been developed as biomaterials in recent years. For example U.S. Patent No. 6,331,312 issued to Lee et al., discloses an injectable calcium phosphate based composite useful as a bone filler and cement. The disclosed material is bio-resorbable and is designed for use in the repair and growth promotion of bone tissue as well as the attachment of screws, plates and other fixation devices. Lee's composition does not expand while setting and is not well suited for attachment of soft tissues, like ligaments, to bone. Lee's invented composition is not believed to promote significant new bone formation.

Many existing calcium phosphate based fillers and cements have high molar ratios of Ca to P making them poorly reabsorbable. Furthermore, a recent FDA release warns of serious complications from the use of existing calcium phosphate based bone fillers in treating compression fractures of the spine (FDA Public Health Web Notification, "Complications Related to the Use of Cement and Bone Void Fillers in Treating Compression Fractures of the Spine," originally published Oct. 31, 2002, updated, May 27, 2004.) Generally, current calcium phosphate cements lack the characteristic of a successful bio-adhesive.

Prior art bio-composites or bio-polymers provides a means for enhancing adhesion to bone and existing structures aside from the chemical adhering aspects of the mixture. As such, fasteners, (such as screws or clamps) often are utilized to hold the physiological structures until the mixtures can cure. Often these fasteners are not biodegradable and can lead to post-operative complications. A few absorbable fixation devices have been developed to diminish post-operative complication including polycarprolactone and various calcium phosphate glass enhanced substances. However, these materials exhibit rapid decline in mechanical strength after their initial application.

A variety of materials have also been developed as bone graft materials. Traditional approaches to bone stimulation include allograft and autograft procedures as well as various ceramic and polymer based bone graft substitutes. Recent advancements include the use of recombinant growth factors like bone morphogenetic protein (BMP) to encourage bone formation.

While existing commercial bio-materials can fill bone defects and/or attach implants to bone, none of the currently available materials provide a bio-adhesive which can fill voids and fractures and is capable of reattaching soft tissues to bone. Furthermore, there are few if any known bio-materials capable of use as an adhesive and osteoproliferative bone graft without the use of growth factors.

A need exists for a resorbable bio-composition that can be used as a bone filler (bone graft) and/or bio-adhesive. The adhesive should incorporate typical calcium-containing moieties to minimize cost and improve biocompatibility. The adhesive should maintain its workability and ultimately "set" under physiologic conditions including temperature, pH and humidity. The material should be absorbed by the body and replaced with the patient's own bone without any untoward side effects. Also, the adhesive should be applicable to bone, implants, ligaments, and tendons so as to provide both void-filling and fracture repair capabilities, as well as structural support. Finally, the bio-adhesive should confer means to both chemically and mechanically fasten structures in place in vivo.

Inventor has spent years developing bio-materials that overcome the shortcomings of prior art compositions. U.S. Patent No. 6,533,821 issued to instant Inventor teaches such a multi-purpose bio-adhesive.

A need also exists for an Improved multi-purpose bio-material that is osteoproliferative, preferably osteoinductive for use as a multi-purpose bone graft, filler, adhesive, binder, anchor and cement The bio-material should be capable of having a controlled exothermic reaction under about 50°C, should be easy to work with, have open working time and be capable of being easily injected using a syringe.

### Disclosure of the Invention

The present invention describes a multi-purpose bio-meterial that is ideal for use as a bio-adhesive, bone and dental cement, bone filler, bone anchor and bone graft. This multi-purpose bio-adbesive generally comprises: KH₂PO₄ ("MKP"), a metal oxide (i.e. MgO), a calcium containing compound, a sugar (or sugar derivate/replacement) and water. The Invented sugar containing bio-adhesive has demonstrated significant osteoproliferative effects that have initially been shown to be osteoinductive.

According to one aspect of the present invention there is provided an osteoproliferative bio-material composition comprising:
a phosphoric acid or phosphoric acid salt present at between about 20 and 70 dry weight percent;
a metal oxide present at between about 10 and 50 dry weight percent;
a calcium containing compound present at between about 1 and 15 dry weight percent; and
a sugary compound present at between 0.5 and 20 dry weight percent and selected from the group selected from the group consisting of sugars, sugar alcohols, sugar acids, amino sugars, glycosaminoglycans, glycolipids, sugar substitutes-and combinations thereof.

The composite may be-applied to bone-contacting surfaces of implant devices as a bone cement. The material may be applied directly to bone defects acting as a bone filler or bone graft. Alternatively the composite may be used in conjunction with various fixation devices such as screws and plates. The material can act as a delivery system when pharmaceutically active agents are added to the matrix. Advantageously, the present material can be used as a bioabsorbable, bio-adhesive to attach soft tissues (i.e. ligaments) to bone without the need of screws or nonabsorbable fixation devices. A feature of a preferred embodiment is the use of sugar to enhance the adhesive, bio-' adsorption and osteoproliferetive qualities of the material

The present invention provides a bio-adhesive that affects the in-situ repair and adherence of body parts to each other and to adjacent structures. A feature of the present invention is that the adhesive can "set" at physiologic temperatures and pH within a short time (i.e. less than about 15-25 minutes), and can be set within extremely short time (i.e. ∼15 second or less) with the assistance of a laser. Another feature of the invention is that the bio-material expands in-vivo. An advantage of the Invented formulation Is its ability to simultaneously fill bone defects and provide structural support. An advantage is the expandability of the adhesive during setting or curing confers additional mechanical contact between the adhesive and body parts and between body parts and such adjacent structures as manmade materials and biological materials.

The present invention also provides a bone substitute/bone graft as a platform for bone formation. An advantage of the substance is its gradual absorption by the body without rejection or adverse reaction to contacted structures. An significant advantage of one embodiment is the osteoconductive and apparent osteoinductive properties of the substance without the use of growth factors.

Briefly, another embodiment of the invention provides a bio-adhesive comprising a means for attaching objects to bone, a means for enhancing said attachment means; and a means for facilitating In vivo degradation of the bio-adhesive. An advantage of the present invention Is its superior adhesive characteristics including the ability to attach soft tissues (i.e. ligaments and tendons) to bone.

According to another aspect of the invention there is provided an orthopedic kit comprising:
the dry bio-material of any one of claims 1-19; and
an aqueous activator solution.

A feature of one embodiment of the invention is its ability to augment reattachment of soft tissues to bone. Preferably the invented biomaterial is used to reattach soft tissue to bone without the need of screws, plates or other fixation devices.

According to a further aspect of the invention there is provided:
a bio-material according to any one of claims 1-19 for use in a method for promoting hard tissue growth comprising the application to a tissue or a hard tissue defect.

Also described is a method for fastening structures to a bone surface, in-vivo, the method comprising accessing the bone surface through a surgically-induced incision; simultaneously applying a phosphate-containing bio-adhesive to the structures and/or to the bone surface; closing the incision, end allowing the adhesive to expand.

The described multi-purpose bio-material is osteoproliferative, and surprisingly osteoinductive. The bio-material is capable of having a controlled exothermic reaction under about 50°C, is easy to work with, has an open working time, and be capable of being easily injected using a syringe.

The described invention is also a useful multi-purpose composition. The invented composition can be used In a variety of ways including but limited to: a coating, fire-retardant, general binder matrix, cement, and refractory. The composition has excellent fire and flame resistance, strong compressive strengths, and excellent adhesive qualities.

### Definitions

"*Osteoconductive*" is the ability of material to serves as a scaffold for viable bone growth and healing.

"Osteoinductive" refers to the capacity to stimulate or induce immature bone cells (or connective tissue) to grow, mature and differentiate into bone, forming healthy bone.

"Biocompatible" refers to a material that elicits no significant undesirable response in the recipient

"*Bioresorbable*" is defined as a material's ability to be resorbed in-vivo through bodily processes. The resorbed material may be used the recipients body or may be excreted.

"Prepared Cells" are defined as any preparation of living cells including but not limited to tissues, cell lines, transformed cells, and host cells. The cells are preferably autologous but can also be xenogeneic, allogeneic, and syngeneic

### Brief Description of the Drawings

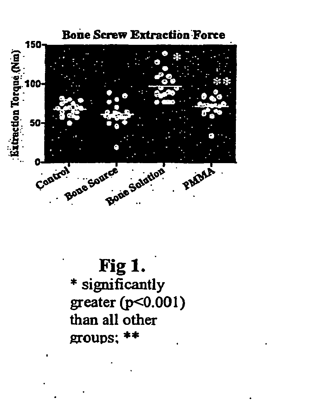

FIG. 1 is a graph of extraction torque results illustrated that the present MgO-MKP-sugar-based product (Bone Solutions) had significantly (p<0.001) greater extraction torque (mean 97.5+/-17.7 Nm) than control, Ca-based product and PMMA. PMMA had significantly (p<0.05) greater extraction torque than Ca-based product.

### Best Mode for Carrying Out the Invention

The invention provides a bio-material for in-situ (i.e. in vivo) attachment of biological structures to each other and to manmade structures. The bio-adhesive also facilitates the repair of bone, ligaments, tendons and adjacent structures. Also provided is a bone substitute for surgical repair. The invented formulation is usable at a myriad of temperatures, pH ranges, humidity levels, and pressures. However, the formulation is designed to be utilized at all physiological temperatures, pH ranges, and fluid concentrations. The mixture typically is injectable, prior to setting and exhibits neutral pH after setting. It is absorbed by the host over a period of time.

The mixture is particularly useful in situations (such as plastic surgery) whereby the use of metallic fasteners and other non-bioabsorbable materials are to be assiduously avoided. The material also is useful when a certain amount of expansion or swelling is to be expected after surgery for example in skull surgeries. It is a good platform for bone-formation. The material can be also used as an anchoring device or grafting material

Generally, the bio-adhesive is derived from the hydrated mixture which comprises: KH₂PO₄, a metal oxide, sugar and a calcium containing compound. Exemplary formulations include the following:

| Formulation I * | |
|---|---|
| Potassium phosphate (i.e. KH₂PO₄) | 61% |
| MgO (calcined) | 31% |
| Ca₁₀(PO₄)₆(OH)₂ | 4% |
| Sucrose C₁₂H₂₂O₁₁ (powder) | 4% |

| | |
|---|---|
| *All values are weight percentages | |

Water is added up to about 40 weight percent of the formulation, preferably between 22-25 weight percent.

| Formulation II* | |
|---|---|
| KH₂PO₄ | 54% |
| MgO (calcined) | 33% |
| Calcium-containing compound | 9% (whereby the compound is Ca₁₀(PO₄)₆(OH)₂) |
| Sucrose C₁₂H₂₂O₁₁ (powder) | 4% |

| | |
|---|---|
| *All values are weight percentages | |

Water is added up to about 40 weight percent of the formulation, preferably between about 22-25 weight percent.

| Formulation III* | |
|---|---|
| KH₂PO₄ | 44% |
| MgO (calcined) | 44% |
| Calcium-containing compound | 8% (whereby the compound is Ca₁₀(PO₄)₆(OH)₂ or CaSiO₃, |
| Sucrose C₁₂H₂₂O₁₁ (powder) | 4% |

| | |
|---|---|
| *All values are weight percentages | |

Water is added up to about 40 weight percent of the formulation, preferably between about 36-38 weight percent.

| Formulation IV* | |
|---|---|
| KH₂PO₄ | 45% |
| MgO (calcined) | 45% |
| Calcium-containing compound | 9% (whereby the compound is Ca₁₀(PO₄)₆(OH)₂, CaSiO₃, or combinations thereof) |
| Sucrose C₁₂H₂₂O₁₁ (powder) | 1% |

| | |
|---|---|
| *All values are weight percentages | |

Water is added up to about 40 weight percent of the formulation, preferably between 22-25 weight percent.

| Formulation V | |
|---|---|
| KH₂PO₄ | 45% |
| MgO (calcined) | 45% |
| Ca₁₀(PO₄)₆(OH)₂ | 8% |
| Sucralose | 2% |

| | |
|---|---|
| *All values are weight percentages | |

Water is added up to about 40 weight percent of the formulation, preferably between 22-25 weight percent.

| Formulation VI | |
|---|---|
| KH₂PO₄ | 61% |
| MgO (calcined) | 32% |
| Ca₁₀(PO₄)₆(OH)₂ | 4% |
| Dextrose | 1.5% |
| a- Ca₃(PO₄)₂ | 1.5% |

| | |
|---|---|
| *All values are weight percentages | |

Water is added up to about 40 weight percent of the formulation, preferably between 22-25 weight percent.

| Formulation VII | |
|---|---|
| KH₂PO₄ | 50% |
| MgO (calcined) | 35% |
| Ca₁₀(PO₄)₅(OH)₂ | 7% |
| ß- Ca₃(PO₄)₂ | 3% |
| Dextrose | 5 |

| | |
|---|---|
| *All values are weight percentages | |

Water is added up to about 40 weight percent of the formulation, preferably between 22-25 weight percent.

| Formulation VIII | |
|---|---|
| KH₂PO₄ | 61% |
| Metal oxide | 32% (wherein the metal oxide is MgO, Ca, FeO or combination thereof), |
| Ca₁₀(PO₄)₈(OH)₂) | 6% |
| Sugar | 1% |

| | |
|---|---|
| *All values are weight percentages | |

Water is added up to about 40 weight percent of the formulation, preferably between 22-25 weight percent.

| Formulation IX | |
|---|---|
| KH₂PO₄ | 54% |
| Phosphoric Acid | 4% |
| Metal oxide | 32% (wherein the metal oxide is MgO, ZrO, FeO or combination thereof), |
| Ca₁₀(PO₄)₈(OH)₂) | 7% |
| Sucrose | 3% |

| | |
|---|---|
| *All values are weight percentages | |

Water is added up to about 40 weight percent of the formulation, preferably between 22-25 weight percent

| Formulation X | |
|---|---|
| KH₂PO₄ | 45% |
| MgO (calcined) | 45% |
| Ca₁₀(PO₄)₆(OH)₂ | 10% |

Water is added up to about 40 weight percent of the formulation, preferably between 22-25 weight percent.

While the above formulations and weight percents are the most preferred proportions, a range of dry constituents can also be used. The range for the phosphate (i.e. MKP) is between 20-70 weight percent, preferably between about 40-65 weight percent. In some situations it maybe preferable to use the phosphate at a range between about 40-50 weight, while In others in may be preferably to use a range of about 50 and 65.

The range for the metal oxide (i.e. MgO) is between 10-50, and even more preferably between 30-50 weight percent. In some situations It maybe preferable to use between about 35 and 50 weight percent.

Calcium containing compounds can be added in various weight percentages. The calcium containing compound(s) is added at about 1-15 weight percent

Sugars (and/or other carbohydrate containing substances) are present at weight percent between 0.6 and 20, preferably about 0.5-10 weight percent of the dry composition.

Water (or another aqueous solution) can be added in a large range of weight percents generally ranging from about 15-40 weight percent

For some embodiments (i.e. formula III) it has been found that adding water at a weight percent of about 37 weight percent produces a creamy textured material that is extremely easy to work with has excellent adhesive properties and is easily injectable through a syringe.

It is important to note that these are exemplary weight percents and that the ranges may vary with the addition of various fillers, equivalents and other components or for other reasons.

A salient feature of the present invention is the ratio between MKP (MKP equivalent, combination, and/or replacement) and the metal oxide. A preferred embodiment has a weight percent ratio between MKP and MgO between about 4:1 and 0.5:1, more preferably between approximately 2:1 and 1:1. In such a preferred embodiment the Inventor surmises that the un-reacted magnesium is at least partly responsible for the in vivo expandability characteristics of the bio-adhesive.

Specifically the metal oxide (i.e. magnesium oxide) reacts with water and serum and In and around the living tissue to yield Mg(OH)₂ and magnesium salts. It has been found that one embodiment of the material generally expands to between 0.15 and 0.20 percent of volume curing curing in moisture. The expansion of the material is believed to increase the adhesive characteristics of the material. For example, the disclosed material has been shown to effectively attach soft tissues like ligaments to bone, the expansion of the material improving adhesion through mechanical strength.

MgO is the preferred metal oxide (metal hydroxide or other equivalent), however, other oxide and hydroxide powders can be utilized in place of or in addition to MgO, including but not limited to: FeO, Al(OH)₃, Fe₂O₃, Fe₃O₄, ZrO, and Zr(OH)₄, zinc oxides and hydroxides, calcium oxide and hydroxides and combinations thereof.

MKP is preferred, but for some applications other compounds may be substituted for (or added to) MKP, including but not limited to: phosphoric acid and phosphoric acid salts like sodium, aluminum phosphate, mono-ammonium phosphate and di-ammonium phosphate.

### Calcium-Containing Compound

A calcium containing compound is essential to the invention as it increases both the bio-compatibility and bio-absorption of the biomaterial. The calcium compound(s) can be selected from a variety of biocompatible calcium containing compounds including but not limited to tricalcium phosphates. Suitable tricalcium phosphates include a-Ca₃(PO₄)₂, ß-Ca₃(PO₄)₂, and Ca₁₀(PO₄)₆(OH)₂.

In general, suitable calcium containing compounds include but are not limited to: tricalcium phosphates, biphasic calcium phosphate, tetracalcium phosphate, amorphous calcium phosphate ("ACP"), CaSiO₃, oxyapatite ("OXA"), poorly crystalline apatite ("PCA"), octocalcium phosphate, dicalcium phosphate, dicalcium phosphate dihydrate, calcium metaphosphate, heptacalcium metaphosphate, calcium pyrophosphate and combinations thereof.

Preferred calcium containing compounds include: tricalcium phosphates, ACP, dicalcium phosphate, dicalcium phosphate dihydrate and combinations thereof.

a-Ca₃(PO₄)₂, ß-Ca₃(PO₄)₂, and Ca₁₀(PO₄)₆(OH)₂, equivalents and combinations thereof, being the most preferred. A preferred tricalcium phosphate is a pharmaceutical or food grade tricalcium phosphate manufactured by Astaris (St. Louis, MO).

Calcium containing compounds increase the bio-compatibility and bioabsorption of the bio-adhesive. However, calcium containing compounds vary in their degrees of bioabsorption and biocompatibility. Some characteristics even vary within the various tricalcium phosphate compounds.

It may be advantageous to combine various calcium containing compounds to manipulate the bio-compatibility and bioabsorption characteristics of the material. For example Ca₁₀(PO₄)₆(OHk (HA") is stable in physiologic conditions and tends to be relatively poorly absorbed while ß-Ca₃(PO₄)₂ is more readily absorbed. The two can be combined (i.e. bi-phasic calcium phosphate) to form a mixture having characteristics somewhere between HA and ß-Ca₃(PO₄)₂. A number of calcium containing compound combinations can be envisioned.

### Sugars, Sugar Substitutes, Sweeteners, Carbohydrates and Equivalents

A salient aspect of a preferred embodiment is the incorporation of at least one sugar or sugar like-substance to the bio-material matrix. Inventor discovered that sugar containing bio-materials have significant osteoproliferative properties as well as enhanced adhesive capabilities. It is believed that a sugar like sucrose may be replaced or supplemented with other sugars and sugar related compounds.

Suitable sugars or sugar related compounds include:
sugars, sugar derivatives (i.e. sugar alcohols, natural and artificial sweeteners (i.e. acesulfame-k, alitame, aspartame, cyclamate, neohesperidine, saccharin, sucralose and thaumatin), sugar acids, amino sugars, glycosaminoglycans, glycolipids, sugar substitutes including sugar substitutes like sucralose (i.e. Splenda®, McNell Nutritionals LLC, Ft Washington, PA), com syrup, honey, starches, and other carbohydrate containing substances.

Exemplary sugars include but are not limited to: sucrose, lactose, maltose, cellobiose, glucose, galactose, fructose, dextrose, mannose, arabinose, pentose, haxose. Preferably the sugar additive is a polysaccharide, more preferably a disaccharide like sucrose.

One preferred additive is sugar combined with a flow agent like starch. An exemplary additive is approximately 97 weight percent sucrose and 3 weight percent starch.

The sugar compound, like the other components, can be in a variety of forms including but not limited to dry forms (i.e. granules, powders etc.), aqueous forms, pastes, and gels. It may prove preferable to use a powdered.

The Inventor has shown that the invented sugar contalnlng bio-material possess surprisingly good adhesive qualities. In fact, the invented composition outperformed current state of the art materials. (discussed below, See Example ! and III). It is believed that the sugar improves the physical (and possibly the chemical) bonding of the cement to objects. The improved adhesion of sugar containing phosphate cements is particularly well suited for attachment of soft tissue like ligaments and tendons to bone without the need for intrusive non-absorbable devices like screws and pins. The elimination of non-absorbable devices reduces post-operative complications and preferably promotes bone growth around the repaired site.

Surprisingly and unexpectedly, it was discovered that a sugar containing composition greatly enhanced formation of new bone. It is believed that the sugar and other compounds of the composition provide near ideal conditions for new bone formation. This assertion is supported by surprising and unexpected test results shown in Example II.

### Bone Graft Material

In one embodiment the composition of present invention provides a bone substitute and a platform for bone formation. An advantage of the substance is its gradual absorption by the body without rejection or reaction to contacted structures. A further advantage of the invented composition is its significant osteaproliferative properties. In fact, in studies the invented composition enhanced bone formation to such a surprising degree, so much so that it Is believed that the composition may also be osteoinductive which is completely unexpected and unprecedented for a multi-purpose biomaterial without the use of growth factors. The bio-material is also believed to have micro and macro pores.

### Additional Embodiments

The formulations disclosed herein may incorporate additional fillers, additives and supplementary materials. The supplementary materials may be added to the bio-material in varying amounts and in a variety of physical forms, dependent upon the anticipated use. The supplementary materials can be used to alter the bio-material in various ways.

Supplementary materials, additives, and fillers are preferably biocompatible and/or bioresorbable. In some cases it may be desirous for the material to be osteoconductive and/or osteoinductive, as well. Suitable biocompatible supplementary materials include but are not limited to: bioactive glass compositions, calcium sulfates, coralline, polyatic polymers, peptides, fatty acids, collagen, glycogen, chitin, celluloses, starch, keratins, nucleic acids, glucosamine, chondroitin, and denatured and/or demineralized bone matrices. Other suitable supplementary materials are disclosed In U.S. Patent No. 6,331,312 issued to Lee and U.S. Patent No. 6,719,992 issued to Constanz.

In another embodiment of the invention the bio-material contains a radiographic material which allows for the imaging of the material In vivo. Suitable radiographic materials include but are not limited to barium oxide and titanium.

The bio-material described herein may prove ideal for creating bioresorbable Implants and devices which can be resorbed by the body overtime, reducing complications while promoting bone reformation. The bio-material can also be used to coat various implant parts.

In yet another embodiment the invented bio-material contains a setting retarder or accelerant to regulate the setting time of the composition. Setting regulators are preferable biocompatible. Suitable retarders include but are not limited to sodium chloride, sodium fluosilicate, polyphosphate sodium, borate, boric acid, boric acid ester and combination thereof.

A preferred retarder composition comprises: a sugar (sucrose) and boric acid in a weight percent ratio of between 0.5:1 and 1:0.5, preferably at a ratio of Approximately 1:1. This setting regulators is preferably added at less than 5 weight % of the dry binder matrix.

The disclosed bio-material may also be prepared with varying degrees of porosity. Controlling porosity can be accomplished through a variety of means including: controlling the particle size of the dry reactants, and chemical and physical etching and leaching. A preferred embodiment increases porosity of the bio-material by addition of 1-20 weight percent of an aerating agent, preferably about 1-5 weight percent. Suitable aerating agents include but are not limited: carbonates and bicarbonates such as: calcium carbonate, sodium carbonate, sodium bicarbonate, calcium bicarbonate, baking soda, baking powder, and combinations thereof.

The biomaterial may be used as delivery system by incorporating biologically active compounds into the bio-material (i.e. antibiotics, growth factors, cell etc.). A porous bio-adhesive increases the effectiveness of such a delivery system.

Cationic antibiotics, especially aminoglycosides and certain peptide antibiotics may be most desirable when incorporating drugs into the blo-material. Suitable aminoglycosides include but are not limited to: amikacin, butirosin, dideoxykanamycin, fortimycin, gentamycin, kanamycin, lividomycin, neomycin, netilmicin, ribostamycin, sagamycin, seldomycin and epimers thereof, sisomycin, sorbistin, spectinomycin and tobramycin. Using inorganic salts like sulfates, phosphates, hydrogenphosphates maybe preferable, sulfates being the most preferable. Further Information about using antibiotics and growth factors in bio-materials can be found in U.S. Patents No. 6,485,754, issued to Wenz. Growth factors include but are not limited to growth factors like transforming growth factor TGF-ß.

The disclosed bio-material composition may also be seeded with various living cells or cell lines. Any known method for harvesting, maintaining and preparing cells may be employed. See U.S. Patents Nos: 6,719,993 issued to Constanz, 6,585,992 issued to Pugh and, 6,544,290 issued to Lee.

One embodiment of the invention has been shown to be extremely useful as a scaffold for hard tissue growth and possibly soft tissue growth as well. In addition, tissue-producing and tissue-degrading cells may be added to the composition included but not limited to: osteocytes, osteoblasts, osteoclasts, chondrocytes, fibroblasts, cartilage producing cells, and stem cells. Methods of isolating and culturing such cells are well known in the art.

The invented composition can incorporated into an orthopedic kit comprising: the material (MKP, metal oxide, calcium containing compounds etc.) in dry form, an activator solution (water or other aqueous solution), and any medical devices (i.e. syringes, knives etc.), implants, or other agents needed during an operation using the invented composition. The material and activator solution will preferably be present in a predetermined, optimized ratio. Other embodiments of such an orthopedic kit can also be envisioned. The biomaterial and other kit components are preferably sterilized by techniques well known in the art.

### Substance Preparation

A metal oxide powder is a salient ingredient in the invented mixture. Optionally, the oxide is subjected to a calcinated process. Calcination durations and temperatures are determined empirically, depending on the final characteristics and setting times desired. Generally, however, calcination temperatures of up to 1300°C for up to several hours are typical.

After calcination, the oxide powder is mixed with MKP, a calcium containing compound, and sugar. One method for sizing and homogenizing the various powders is via vibratory milling. Another homogenization method utilizes a ribbon mixer wherein the particles are ground to a fine size. Dry compounds are disclosed herein, however, aqueous versions (or other forms i.e. gels etc) of some of the bio-materials components can also be utilized. Generally, pharmaceutical grade compounds are utilized. Sterilization of the various components may be required using sterilization techniques known in the art.

Upon homogenization wherein all of the constituents are contained in a dry homogeneous mixture, water (or other aqueous solution) is generally added up to about 40% of the weight of the resulting slurry although the amount of water can be adjusted to form a blo-materlal of varying viscosity. The slurry is mixed for between 1-10 minutes depending upon conditions. Mixing can be achieved by a variety of techniques used in the art including hand and electric fixing. See, U.S. Patent 6,533,821 issued to present Inventor for further details.

The bio-material can be created in injectable, paste, puddy and other forms. The slurry is produced at the user site. The consistency of the material can be manipulated by varying the amount of water added to the dry mixture. Increasing the water content generally Increases the flowability while decreasing the water content tends to thicken the slurry. The material can be prepared in a myriad of forms.

- Working times can be increased or decreased by varying the temperatures of bio-material components. Higher temperature components tend to react and set quicker than cooler components. Thus regulating the temperature of the water (or other reactants) can be an effective way.to regulate working time.

Bonding occurs primarily between the adhesive and bone. However, the adhesive also bonds to itself, or to soft tissue. The Inventor has found that the use of a phosphoric acid instead of water increases the bonding strength of the material. The molarity of the phosphoric acid can vary, as long as the eventual pH of the slurry Is not hazardous to the patient, or contraindicative to healing. Generally, a slurry pH of between 6 and 8 is appropriate, however other slurry pHs may be employed depending on desired results.

### Attachment

The attachment of the bio-adhesive to various structures can be accomplished in a number of ways including but not limited to: Injection, spraying, and other application means. The attachment means will vary according to the desired application and the form of the adhesive. One exemplary method is described in instant inventors U.S. Patent Application No, 6,533,821.

### Example I

An experiment comparing the adhesive qualities of a prior art bone filler (NORIAN® Skeletal Repair System, Paoli, PA) and a preferred embodiment of the present bio-adhesive having the weight percent formula: 54% MKP, 33% magnesium oxide, 9% Ca₁₀(PO₄)₆(OH)₂, and 4% Sucrose mixture (the sugar mixture being 97% sugar and 3% starch).

The goal of the study was to determine if an injectable MgO-MKP-sugar based formulation of the present invention had adhesive properties for bone to bone and tendon to bone using clinically relevant models. Biomechanical studies were performed using a canine cadaver model of anterior cruciate ligament repair and femur fracture. Tissue adhesion was quantified with mechanical pull-out and three-point bending studies. Sixteen knee joints with femurs and Achilles tendons from 8 mid-sized dogs were harvested and three tissue contructs for testing were prepared.

*ACL Model: A) Bone to Bone.* Bone-patellar ligament grafts were cut and the patella bone press-fit into a 7mm diameter bone tunnel in the femur at the ACL footprint to mimic human ACL reconstruction. The ligament end served as the anchor for pull out mechanical testing. *B) Tendon to Bone.* Achilles tendon grafts were placed through a 7mm diameter tibial bone tunnel initiated at the ACL footprint and exiting the lateral tibial cortex to mimic human ACL reconstruction. Anchoring screws or sutures were not used to augment these repairs. Treatment groups were: 1) Press-fit (Control; n=16); 2) Calcium based injectable formulation (n=8) (Negative paste control) (Norian® Skeletal Repair System- Synthes, Paoli, PA); 3) MgO-MKP-sugar based bioadhesive. Limbs were paired for groups 2 and 3. Product was prepared and injected into the bone defects surrounding the bone or tendon grafts in the bone tunnels and allowed to cure overnight. Grafts were mechanically tested in tension for peak load to failure at 1 mm/sec.

*Fracture Model:* A 1cm long oblique osteotomy was made in the midshaft of the femur diaphysis and four materials tested to hold the fracture in reduction: 1) Blood clot (freshly clotted equine blood); 2) cyanoacrylate glue (Ross Super Glue Gel- Ross Products, Columbus, OH); 3) Calcium based injectable formulation (Norian® Skeletal Repair System- Synthes, Paoli, PA); 4) MgO-MKP-sugar based injectable formulation. Additionally, four intact femurs were tested to failure. Groups 3 and 4 were tested in paired limbs. Groups 1 and 2 were tested in paired limbs; one half before and one half after application of the paste products in groups 3 and 4. First tested products were readily removed by scraping. Injectable pastes and cyanoacrylate were applied liberally to the fractured bone ends, held together for 15 minutes until hardened, and allowed to cure overnight. Blood clot was applied immediately before testing. Femurs were tested in 3-point bending under displacement control at 0.1 mm/sec for peak load to failure. Stiffness and stress to failure were calculated from the slope of the linear portion of the load deformation curve and after estimation of bone area at the fracture with calipers. Fractures which fell apart before testing were recorded as 0 N to failure.

Data in the ACL model were analyzed with the paired Student's t-test for calcium vs magnesium formulations and for press fit vs formulation. Data in the fracture model were analyzed with a 1-factor ANOVA for treatment group. Significance was set a p<0.05.

RESULTS: In the ACL model, both the calcium based formulation and the MgO-MKP-sugar based formulation had significantly greater pull out force than press-fit (friction) within the tunnel for both patellar bone and Achille's tendon (p<0.004). The MgO-MKP-sugar based formulation had the greatest adhesive properties, significantly greater than the calcium based formulation for both bone (2.5-fold;p<0.0) and tendon (3.3-fold;p<0.0). (Table 1)

In the fracture model, blood clot and calcium based formulation had no adhesive properties (0 N load to failure) in all specimens. Blood clot was unable to hold the two ends of the femur in apposition. The calcium based product held the femur ends in apposition, but separation occurred prior to testing. MgO-MKP-sugar based formulation and cyanoacrylate failed at significantly greater loads (p<0.0001) and cyanoacrylate failed at significantly greater loads (127 N; p<0.01) than the MgO-MKP-sugar based formulation (37.7 N). Intact femurs failed at much greater loads with any bone adhesive achieving less than 10% of original bone strength.

**Table 1. ACL Model - Peak Mean (+/- SEM) Tensile Load (N) to Failure.**

| **Groups** | **Press-fit** | **Ca-based Formulation (Norian®)** | **MgO-MKP-sugar based Formulation** |
|---|---|---|---|
| **Bone-Bone** | 41.6 +/- 16.8^{a} | 427.7 +/103.9^{b} | 1025.6 +/-118.2^{c} |
| **Tendon-Bone** | 12.9 +/-0.03^{a} | 101.6 +/- 23.1^{b} | 338.2 +/-69.9^{c} |

**Table 2. Mean (+/- SEM) Biomechanical Properties to Failure in Femur Osteotomies Repaired with Potential Bone Glues**

| **Groups** | **Peak Load (N)** | **Peak Stress (N/mm2)** | **Stiffness (N/mm)** |
|---|---|---|---|
| **Blood Clot** | 0 +/- 0 | 0 +/-0 | 0 +/-0 |
| **Ca-based Formulation (Norian®)** | 0 +/-0 | 0 +/-0 | 0 +/-0 |
| **MgO-MKP based Formulation** | 37.7 +/-27.4 | 0.09 +/- | 148.7 +/- |
| **Cyanoacrylate** | 127.0 +/- | 0.3+/- | 783 +/- |
| **Intact Femur** | 1455.8 +/- | 4.18+/- | 666.8 +/- |

In bone and tendon pullout from a bone tunnel, paste formulations provide some adhesion due to cement properties (ie hardened filler). However, the MgO-MKP-sugar based formulation had additional and substantial adhesive properties of over 1000 N in bone that should exceed forces put on the construct in vivo. In femur fracture reconstruction, the MgO-MKP-sugar based formulation provided bone adhesion, but not as great as our nonbiodegradable positive control glue. Repaired construct strength was still < 10% of intact femur strength, but may provide fragment containment and osteoconduction.

A biodegradable MgO-MKP-sugar based, injectable formulation adhered bone and tendon within bone tunnels sufficiently to significantly augment, or potentially be used independently, in ACL reconstructions. Adhesion of bone ends may be sufficient to contain fracture fragments in comminuted fracture repair and may be useful if osteoconduction and biodegradation profiles complement fracture healing as anticipated.

### Example II OSTEOPROFLIFERATIVE RESULTS: Formula II

### ANIMALS:

| | |
|---|---|
| Species/breed: | Equine/Mixed Breed |
| Initial age: | A minimum of 3 years maximum of 20 years at start of acclimatization |
| Initial weight: | Approximately 800-1200 kg at acclimation |
| Sex: | geldings, mares |
| Identification of animals: | Individual neck collar, ear tag or halter tag |
| Pretreatment: | Vaccinations: Eastern, Western Encephalitis, Influenza; West Nile Virus and tetanus. De-wormed post arrival at Ohio State Finley Research farm. Animals will have had no previous compound exposure. |

### SITE DESCRIPTION:

This study will be conducted at the Ohio State University Alice Finley Memorial farm (Finley farm) and the Veterinary Teaching Hospital (VTH). Evaluation will take place at the Veterinary teaching hospital. The facility's animal accommodations, laboratory support areas, record keeping, and anticipated compliance are to be satisfactory to meet the requirements of this protocol.

### MANAGEMENT:

| | |
|---|---|
| Floor space per animal: | Animals will be housed in box stalls for the duration of the study. |
| Feeding and watering method: | Hay and grain is fed twice/day. Water will be provided *ad libitum.* |
| Housing: | Bedded box stalls at the Finley farm or VTH. |
| Environmental control: | Finley farm box stalls are in a barn that is not temperature regulated. VTH box stalls are sheltered in a building and are temperature regulated. |
| Feed: | Approximately 3 lbs. grain/animal/day. Hay will be offered at approximately 15 lbs twice daily and more as necessary. |
| Water: | Water will be checked daily and cleaned if necessary. |

### DESIGN:

Experimental Study; Nested Paired Design; Each horse serves as its own control. Horses, limb, and medial or lateral splint bone are assigned in a controlled block design. Eight horses, bilateral Mtll and MtlV fractures (24 splint bones). One medial and one lateral splint (MtII and MTIV) will be treated with MgO-MKP-sugar injectable formulation (n=16). The contralateral splint will be injected with either Calcium-based injectable formulation [Comparative treatment] or receive no injection (Untreated control) Table 3 the result is 4 groups of 8 limbs each: 1) Untreated Natural healing (control), 2) Calcium-based nonadhesive injectable product [Treatment comparison], 3) Magnesium-based adhesive injectable test product

**Table 3. signment of metatarsi (splints) to treatment groups (n=8 per group)**

| HORSES | METATARSAL II -TREATMENT | | METATARSAL IV-TREATMENT | |
|---|---|---|---|---|
| | None | Bone Solutions | Bone Scource | Bone Solutions |
| | | Product | Product | Product |
| | | MgO-MKP-sugar | Ca-based | MgO-MKP-sugar |
| 340 | X - right | X - left | X - left | X - right |
| 352 | X - left | X - right | X - right | X - left |
| 354 | X - right | X - left | X - left | X - right |
| 362 | X - left | X - right | X - right | X - left |
| 365 | X - right | X - left | X - left | X - right |
| 366 | X - left | X - right | X - right | X - left |
| 369 | X - right | X - left | X - left | X - right |
| 377 | X - left | X - right | X - right | X - left |

### PROCEDURE:

Inclusion Criteria: Horses (aged 3-20 yrs) must be healthy on physical examination and complete blood count, and be sound with no palpable or radiographic abnormalities of the metatarsus.

Blinding: Splint and limb assignments will be recorded. All radiographic, qCT, biomechanical testing and histomorphology will be performed with samples coded in a blinded fashion.

Fracture Model - Fractures (Mt (Splint) II and Mt (Splint) IV) will be performed under general anesthesia at day 0. Horses will be administered procaine penicillin (22,000units/)<g) intramuscularly and gentamicin (6.6 mg/kg) intravenously 30 minutes prior to anesthesia. Horses will be sedated with xylazine HCl (1 mg/kg), induced with ketamine (2mg/kg) and maintained in dorsal recumbency on isoflurane and oxygen to effect. The splint bones are directly under the skin at the locations for these bone defects. After aseptic preparation, small 2-cm incisions will be made over the smooth palpable surface of the splint bones; 15 cm distal to the palpable tarsometatarsal joint A curved spatula is placed under the splint bone and a nitrogen-driven oscillating bone saw used to create a 3-piece fracture containing a triangular fragment [90°. 1.5-cm arm]. The bone saw removes a 1 mm width of bone. The incisions are flushed liberally with saline to remove bone dust and dried. Bleeding will be arrested on the bone surface by pressure or radiofrequency cautery. The triangular piece of bone will be placed back into the parent defect according to assignment. If the bone is assigned to receive injectable paste, it will be mixed according to manufacturer's recommendations, ∼0.5ml will be placed onto the cut bone surface and the triangular piece glued back into place. The fragment will be press fit into place for 30 minutes to assure curing or permit blood clot in the control specimens. A layered closure of the incision will be performer, a sterile bandage applied and horses recovered. Sterile bandages are maintained for 2 weeks.

Material Preparation: Bone Solutions product (MgO-MKP-sugar based) and Bone Source product (Ca based) were mixed with a metal spatula just prior to application in order of Table 3 and applied into the fracture gap with a metal spatula. Both products were applied after 2 minutes of mixing and reapplied as needed to position sufficient material into the fracture bed.

### OUTCOME ASSESSMENTS:

Clinical Assessments - Horses will be monitored daily for clinical signs of any reaction to the procedures or therapy. Rectal temperature (T), heart rate (HR) and respiratory rate (RR) will be recorded daily for 1 week following surgery and following injections and then weekly until termination of the study at 8 weeks.

*Pain -* Horses will be monitored for pain by assessing physical parameters (T, HR, RR), lameness scores (0-5) while in the stall.

*Swelling -* Surgical site swelling will be assessed by score [0-4; 0= no swelling and 1= minimal, 2=mild, 3= moderate, and 4= marked swelling]. Surgical site drainage will be assessed by drainage score of drainage character (color, viscosity) [0-4; 0=no drainage, 1=0-25% of the bandage surface stained with drainage, 2=26-50% of the bandage surface stained with drainage, 3=51-75% of the bandage surface stained with drainage; 4=76-100% of the bandage surface stained with drainage].

*Gait Assessment -* Lameness will be scored 0-5 for each hindlimb at the walk on week - 1,1,3,4,5,6,7, and 8. [0=no lameness, 1=minimal lameness, 2 mild lameness, 3 moderate lameness, 4 marked lameness (only placing part of the foot), and 5 =non-weightbearing lameness.

*Euthanasia -* Horses will be euthanized at 7 weeks within the guidelines of the AAEP by an overdose of intravenous pentobarbital solution after sedation with 500 mg xylazine HCl IV and the distal limbs harvested.

### Fracture Healing (Bone Adhesion and Union) -

*Radiographs -* Oblique radiographs will be taken before fracture and injection, and every other week for 7 weeks until termination. Radiographs will be scored for fracture fragment migration (0=none, 1 =minimal" 2=mild, 3=marked), bone proliferation (0=none, 1=minimal, 2=mild, 3=marked), bone remodeling (0=none, 1=minimal, 2=mild, 3=marked), and fracture closure (0=none, 1=minimal, 2=mild, 3=complete). The width and length of the fracture callus will be measured and calibrated using a radiographic measuring standard included in all films.

*Quantitative Computer Tomography (qCT) -* The metatarsus of the distal limbs will be screened at 1 cm intervals for soft tissue abnormalities associated with the fracture healing process. At and for at least 1cm proximal and distal to the bone defect sites, 1 mm slices will be obtained. Subsequently, Mt IV and MtII will be harvested, cleaned of soft tissue and scanned in cross section in 1 mm slices from the top to the bottom of the callus to determine area, density and mineral content (area x density) of mineralized callus. Each slice will be standardized for x-ray attenuation differences for density measurements by using potassium phosphate standards. After standardization, a calculation will be performed to convert potassium phosphate region of interest (ROI) to ash density (mg/mm3). Tracings of the ROI will be performed on cross section views from bone at the healed fracture site for bone area (amount of bone), density of bone in the healing fracture, and density of bone in the callus. Splints will be mechanical tested immediately after qCT.

*Mechanical Testing-Metatarsal* II and IV ends will be secured in grips tested quasi-statically to failure in 3-pt bending (1.5 mm/sec) using a servohydraulic materials testing system. The bones will be positioned in the jig to ensure appropriate and bending for both right and left sides. The load/deformation data will be collected and maximum load to failure calculated.

*Histology -* After mechanical testing splint bones will be embedded undecalcified in PMMA, sectioned (10um) in the longitudinal frontal plane [EXACKT system, OSU], stained with Masson's Trichrome, and evaluated for callus composition, maturity, cortical continuity, and fracture bridging. Assessment of tissue type, such as cartilage, fibrous tissue and bone, within the defect will be noted.

Data Analysis_{z} Descriptive statistics will be generated for all outcome variables. A paired t-test will be used to evaluate the effect of MgO-MKP-sugar-based (Mg-based) injectable paste treatment compared to Calcium-based or no treatment on healing for objective data. Scored data will be expressed as median and range and analyzed by Mann Whitney U Rank test Differences will be considered significant at p<0.05.

### FINDINGS AND CONCLUSIONS

Experimental Design: All 8 horses completed the 7 week healing study as per the assignment in Table 3. All horses met the inclusion criteria. Signalments are listed in Table 4. All horses underwent surgery to create the triangular metatarsal fractures and application of the assigned treatment. The fragment was press fit into the parent defect for 30 minutes and materials seemed cured at surgical closure.

**Table 4. Signalment of horses used in this study.**

| Horse # | Breed | Sex | Approximate Age (yrs) | Scale Weight (kg) |
|---|---|---|---|---|
| 340 | Morgan/Standardbred | Female | 9 | 491 |
| 352 | Thoroughbred | Female | 9 | 513 |
| 354 | Standardbred | Female | 17 | 480 |
| 362 | Paint | Female | 8 | 519 |
| 365 | Standardbred | Female | 10 | 528 |
| 366 | Paint | Female | 11 | 534 |
| 369 | Quarter Horse X | Female | 7 | 486 |
| 377 | Quarter Horse X | Female | 6 | 554 |

| | | | | |
|---|---|---|---|---|
| OUTCOME ASSESSMENTS: | | | | |

### Clinical Assessments -

*Pain and Gait -* Horses were not lame at any time point following surgery as estimated by lameness score (median 0, range 0) as per protocol. Physical examination parameters remained within normal limits through out the study.

*Incisional Swelling and Drainage -* There was no difference in swelling postoperatively among the 4 treatment groups and there was no drainage at the incisions at any time point. At termination of the study, only one surgical site had a palpable firm, nonpainful ∼ 2cm enlargement. The interpretation of these data is that the Mg and Ca materials are clinically biocompatible, clinically nonirritating. Clinically evident tissue or bone proliferation did not occur and therefore was not excessive.

*Radiographs-* Radiographs were taken as per protocol before surgery and every other week until the termination of the study. Radiographs were evaluated for fragment gap, presence of material, bone formation, bone remodelling and bone healing. Migration of the fragment was assessed as the distance (mm) from the apex of the fragment to the apex of the fragment bed as a straight line. The MgO-MKP-sugar treatment secured the fragment significantly closer (P<0.05) to the parent fragment bed than either no treatment or Ca-treatment immediately after surgery (week 0). Migration of the fragment did not occur in the Mg- or Ca-treatments until week 4 in MtII or until week 2 in MtIV. The fragment migrated less in the MgO-MKP-sugar-treatment as compared to no treatment at all time points and this was statistically significant for up to 4 weeks. (See appendix for graph and data) Callus formation (bone proliferation at the healing fragment) was estimated from the radiographs by measuring the width and height of the new bone formed around the fragment at its greatest point and multiplying these numbers to estimate area of new bone. New bone callus was significantly greater in the MgO-MKP-sugar-treatment (Mg-treament) than both the Ca-treatment and no treatment in both MtII and MtIV. Significant formation of bone occurred by 4 weeks and persisted through 7 weeks.

Radiodense material could be identified in the gap between the fragment and parent bone on the radiographs of some horses at some time points, particularly the early time points. (See graph in appendix) product was noted of equal frequency and amount to Ca product until week 4 after which less material was noted in general (lower scores but greater in Mg group, and at week 7 only in the MgO-MKP-sugar group.

Bone remodelling around the fragment and parent bone, was significantly greater in the MgO-MKP-sugar -treatment than in the no treatment or Ca-treatment groups.

Bone healing around the fragment and parent bone was greater in the MgO-MKP-sugar -treatment and this was significant (p<0.05) in all weeks compared to no treatment and at weeks 4, 6 and 7 compared to Ca-treatment.

*Euthanasia and Bone Harvest -* Horses were euthanized at 7 weeks postoperatively as outlined by the protocol. Metatarsi and distal limbs were cut off, labelled, stored in plastic and frozen.

### Quantitative Computed Tomography -

Intact limbs and metatarsal bones (4 per horse) were scanned [Picker P Helical CT. Philips Medical Systems for North America, Bothell, WA]after 7 weeks of healing. Intact limbs were scanned in cross section at 1cm slices and each slice evaluated subjectively for dystrophic mineralization of the surrounding soft tissue. No abnormal mineralization was noted including in the suspensory ligament, tendons or surrounding skin. Metatarsal bones were scanned in 1 mm slices in sagittal section from medial to lateral and to include at least 1 cm above the callus to 1 cm below the callus. The central slice of the metatarsal scans that transacted the fragment was selected and a region of interest traced for the gap, the fragment, and the callus. For the regions of interest for the gap, the fragment and the callus, measurements were recorded for density of tissue and size of region. Density measurements were then transposed from potassium phosphate density to ash density using the phantom calculations simultaneously collected with each slice. There was a tendency (p<0.08) for the density within the gap between the fragment and the parent bone to be greater in the MgO-MKP-sugar -treatment when compared to no treatment. There was no difference (P<0.13) in density of the gap comparing Mg and Ca treatment. When taken in concert with the scored data from the radiographs, this likely reflects the presence of material at 7 weeks. (See raw data and tablulated data in appendix) There was no significant difference in density or the size of the fragment between groups. There was significantly greater amount of callus around the healing fragment in the Mg-treatment compared to no treatment (p<0.01) and Mg-treatment compared to Ca-treatment (p<0.02). These data corroborated the radiographic measurements of greater callus. In summary these data show that there was no destruction of the fragment by the materials, no abnormalities in the density of bone formed and that the Mg-treatment significantly increased bone formation at the fragment site. This osteoproliferative effect seen in this model and species is an osteoinductive response to the Mg-product. Further investigation using the highest purity product and standard osteoinduction models will confirm this finding.

*Mechanical Testing -* Bones were failed in 3-pt bending and measurements recorded for peak load to failure (N) and cross sectional diameter (mm). Calculations were made for peak stress to failure (N/mm2). There was no significant differences in the mechanical testing results among any groups. The size and strength of the healed MtIV was significantly greater than MtII. (See appendix for data)

*Histology-* Bones were sectioned in cross section to mimic the plane of the qCT assessments and to see the fragment and surrounding bone is cross section. Material staining brightly was grossly obvious in 6 of the 8 Mg-treated Mt IV bones and 3 or the 8. Mg- treated Mt II bones. Material was grossly apparent in 4 of the 8 Ca- treated Mt IV bones. Histologic evaluation of the specimens revealed that the tissue types adjacent to the fragments and material was fibrous tissue and/or bone. There was no inflammatory cells within this adjacent tissue. There was no granulomatous response (influx of giant cells). Bone was noted to be directly adjacent to the material. The histology data supports the following conclusions. The Mg material is not absorbed and remained adhere to the site for 7 weeks. The Ca material was either absorbed or migrated from the site by 7 weeks in many of the specimens. Both the Ca and Mg material is biocompatible and did not incite an inflammatory reaction. The body did not wall off the materials. Bone or fibrous tissue, the anticipated healing tissue types were abundant and in close proximity to material without effect.

### APPENDIX I-DOSAGE ADMINISTRATION

All animals will receive Bone Source and Bone Solutions Products. Products will be mixed immediately prior to placement, using a spatula, into the bone defect to cover all surfaces of bone. Bone fragments will be held into position for a minimum of 5 minutes and allowed to cure for a minimum of 30 minutes before skin closure. Bleeding will be controlled on the surface of the bone before applying paste or replacing the fragment (untreated control).

### APPENDIX II-PHYSICAL EXAMINATION

Inclusion Criteria:
1. Normal on physical examination form (including lameness). Jog with score of less than 1
2. Palpation of both metatarsi will be acceptable.
3. Acceptable CBC and chemistry profile
4. Acceptable radiographs of both metatarsi.

Physical examinations will be performed by an appropriately experienced veterinarian and will include rectal temperature, evaluation of tongue and gingivitis including capillary refill time, heart rate, respiratory rate, thoracic and GI auscultation, and the assessment of the general physical condition of each animal.

### APPENDIX III-CLINICAL PATHOLOGY

### Hematology, Serum Chemistry will be performed as standard at OSU clinical pathology laboratory

Blood samples will be taken for hematological examination, serum chemistry and plasma drug exposure. Two types of sterile evacuated tubes will be used for blood collection. Tube size will be appropriate for the volume of sample required. A tube with EDTA anticoagulant will be used for hematology, a tube with no anticoagulant will be used for serum collection and a tube with EDTA will be used for plasma drug exposure. All tubes with anticoagulant will be gently inverted after filling.

### EXAMPLE III-ADHESION TO STEEL SCREWS INTO BONE-Formula II

A biodegradable monopotassium phosphate, magnesium [Mg] oxide, tricalcium phosphate, sugar injectable formulation will increase screw extraction torque and surface bonding compared to polymethylmethacrylate [PMMA], calcium [Ca] phosphate or no bone cement.

Bone cements serve as bone void fillers and can cement structures, such as implants into bone. Bone cements are used to secure joint implants into bone cavities', lute plates and screws onto bone², and enhance srcew pullout forces³. Mechanisms of action for enhancing security of the implants in these applications include hardening within the bone cavity and increasing surface contact area. None of the currently available cements (biodegradable or nonbiodegradable) claim to adhere implants to bone, but this property could further enhance the security of implants in bone and reduce micromotion. A MgO-MKP-sugar formulation has demonstrated adhesive properties for bone to bone and tendon to bone,⁴ and may therefore provide adhesion of implants to bone. The specific goal of this study was to determine if a MgO-MKP-sugar (Mg-based) bone cement had adhesive properties to stainless steel screws compared to a Ca-based commercial product and PMMA. Implant security was quantified as peak extraction torque. Material distribution and bonding to the implant was assessed with high-detailed radiography and undecalcified histology. Extraction torque was selected to represent bone-material-implant bonding because interface failure, rather than failure of the material or bone, occurs at the loss of implant security.

METHODS: Sixteen paired radii were harvested from 8 mid-sized dogs. Four holes were drilled, equidistant, from cranial to caudal in the distal diaphysis.⁵ The bones. were secured in a jig and drilled perpendicular to the surface with a 2.5 mm drill bit and the length of the hole measured with a depth gauge. The holes were manually tapped to be filled with a 316L stainless steel cortical bone screw [Synthes, Paoli, Pa] of appropriate length to a torque of 0.706 Nm [Qdriver2 Torque Screwdeiver, Snap-on Inc., Kenosha, WI] according to the following assignments: Gp1-Control, No material; Gp2- Ca-based biodegradable bone filler/cement [Bone Scource; Stryker Inc, Kalamazoo, MI]; Gp3-PMMA [Simplex™P, Stryker Inc., Kalamazoo, MI]; and Gp4- Mg-based biodegradable bone filler/cement [Bone Solutions, Dallas, TX]. Material was prepared and used to fill the assigned holes which were rotated to control for hole position from proximal to distal. In rapid succession, the screws were placed and the material.allowed to cure for 96 hrs.The extraction torque (Nm) for each screw was tested and measured using a Torque Sensor/Load Cell Display [Transducer Techniques Inc. Temecula, CA] connected with a torque wrench during derotation of screws. Peak values were recorded (Nm). Radii were digitally radiographed and the cemented area around each hole measured using an electronic pen [Osirix Medical Imaging Software] and recorded. Screws were reinserted and bones were cut into slabs on either side of the hole, sectioned undecalcified [Exackt System, Zimmer, Warsaw, IND] cranial to caudal, and stained with Masson's trichrome stain. Histologic sections were evaluated qualitatively for interface gap, bone/screw/material contact, and material microscopic appearance. RESULTS:The Mg-based product (Bone Solutions) had significantly (p<0.001) greater extraction torque (mean 97.5+/- 17.7 Nm) than control, Ca-based product and PMMA. PMMA had significantly (p<0.05) greater extraction torque than Ca-based product. (Fig 1) An area of cement around the screw was identifiable in all materials, but significantly greater (p<0.001) in Mg-based product and PMMA than control or Ca-based product [Table 5] and was obvious grossly.

**Table 5. Mean (+/- SEM) area (pixels²) of cement present surrounding screws placed In canine radii.**

| Control | Ca-based Bone Scource | Mg-based-Bone Solutions | PMMA |
|---|---|---|---|
| 0+/-0 | 519 +/-36 | 973 +/-100* | 1309+/-179* |

| | | | |
|---|---|---|---|
| *P<0.001 | | | |

Histologically the Ca-based product was granular, dense, homogeneous with a gap at the Interface. The PMMA was finely granular, homogeneous and in contact at the interface. The Mg-based productwas granular, nonhomogeneous, in direct contact with screw and bone. The material was densely packed at the interface,

DISCUSSION: The Ca-based cement did not provide greater extraction torque on the screw due to separation at the interface. PMMA diffused into the surrounding bone, provided a tight bond at the screw interface, and greater extraction torque than Ca-based cement or control, but is not biodegradable. Mg-based cement diffused into the surrounding bone, provided a tight bond at the screw interface, the greatest extraction torque and is biodegradable. The mechanism of superior adhesion to the implant appeared to include expansion and compression against the surface of screw and bone. CONCLUSION: A biodegradable magnesium injectable cement was superior at securing stainless steel implants in bone.

REFERENCES: 1) Sporer and Paprosky. (2005)36:105;2)Anderson et al. Vet Surg (2002) 31:3;3)Griffon: et al. Vet Surg (2005):34:223;4)Bertone et al. (2005)Trans ORS:1007;5)Linn et al. V.C.O.T. (2001) 14:1-6.

Having described the basic concept of the invention, it will be apparent to those skilled In the art that the foregoing detailed disclosure is intended to be resented by way of example only, and is not limiting. Various alterations, improvements, and modifications are intended to be suggested and are within the scope of the present invention. Additionally, the recited order of the elements or sequences, or the use of numbers, letters or other designations therefore, is not intended to limit the claimed processes to any order except as may be specified In the claims. Accordingly, the invention is limited only by the following claims and equivalents thereto.

## Claims

1. An osteoproliferative bio-material composition comprising:
a phosphoric acid or phosphoric acid salt present at between about 20 and 70 dry weight percent;
a metal oxide present at between about 10 and 50 dry weight percent;
a calcium containing compound present at between about 1 and 15 dry weight percent; and
a sugary compound present at between 0.5 and 20 dry weight percent and selected from the group selected from the group consisting of sugars, sugar alcohols, sugar acids, amino sugars, glycosaminoglycans, glycolipids, sugar substitutes and combinations thereof.

2. The bio-material composition of claim 1, wherein the weight ratio between the phosphoric acid or phosphoric acid salt and the metal oxide is between about 2:1 and 1:1.

3. The bio-material composition of any one of claims 1-2, wherein the phosphoric acid or phosphoric acid salt is selected from the group consisting of KH₂PO_{4,} sodium phosphate, aluminum phosphate, mono-ammonium phosphate and di-ammonium phosphate, and combinations thereof.

4. The bio-material composition of any one of claims 1-2, wherein the phosphoric acid or phosphoric acid salt is KH₂PO₄.

5. The bio-material composition of any of claims 1-4, wherein the metal oxide is selected from the group consisting of MgO, FeO, Al(OH)₃, Fe₂O₃, Fe₃O₄, ZrO, Zr(OH)₄, zinc oxides and hydroxides , calcium oxide and hydroxides and combinations thereof.

6. The bio-material composition of any one of claims 1-4, wherein the metal oxide is MgO.

7. The bio-material composition of any one of claims 1-6, wherein the calcium containing compound is selected from the group consisting of: α-Ca₃(PO₄)₂, ß-Ca₃(PO₄)₂, Ca₁₀(PO₄)₃(OH)₂, tetracalcium phosphate, amorphous calcium phosphate, bi-phasic calcium phosphate, poorly crystalline apatite, oxyapatite, octacalcium phosphate, dicalcium phosphate, dicalcium phosphate dihydrate, calcium metaphosphate, heptacalcium metaphosphate, calcium pyrophosphate and combinations thereof.

8. The bio-material composition of claim 7, wherein the calcium containing compound is a tricalcium phosphate.

9. The bio-material composition of claim 8, wherein the calcium containing compound is selected from the group consisting of α-Ca₃(PO₄)₂, ß-Ca₃(PO₄)₂, Ca₁₀(PO₄)₆(OH)₂.

10. The bio-material composition of claim 8, wherein the tricalcium phosphate is Ca₁₀(PO₄)₆(OH)₂.

11. The bio-material composition of any one of claims 1-2, wherein
the phosphoric acid or phosphoric acid salt is KH₂PO₄;
the metal oxide is MgO; and
the calcium containing compound is a tricalcium phosphate.

12. The bio-material composition of any one of claims 1-11, wherein the sugary compound is selected from the group consisting of: sugars, sugar alcohols, sugar acids, amino sugars, sugar polymers, and combinations thereof.

13. The bio-material composition of claim 12, wherein the sugary compound is a sugar.

14. The bio-material composition as recited in claim 13, wherein the sugary compound is sucrose.

15. The bio-material composition of claim 12, wherein the sugary compound is a polysaccharide.

16. The bio-material composition of any one of claims 1-15, wherein:
the phosphoric acid or phosphoric acid salt is present at between about 40 and 65 dry weight percent;
the metal oxide is present at between about 30 and 50 dry weight percent;
the calcium containing compound is present at between about 1 and 15 dry weight percent; and
the sugary compound is present at between 0.5 and 20 dry weight percent.

17. The bio-material composition of any one of claims 1-15, wherein:
the phosphoric acid or phosphoric acid salt is KH₂PO₄ present at between about 40 and 50 dry weight percent;
the metal oxide is present at between about 35 and 50 dry weight percent;
the calcium containing compound is present at between about 1 and 15 dry weight percent; and
the sugary compound is present at between about 0.5 and 10 dry weight percent.

18. The bio-material composition as recited in any of claims 1-17, wherein the sugary compound is present at between 0.5 and 10 dry weight percent.

19. The bio-material composition of any one of claims 1-18, wherein the composition is osteoinductive.

20. The bio-material composition of any one of claims 1-19, further comprising an antibiotic, a growth factor, or a living cell.

21. The bio-material composition of any one of claims 1-20, further comprising an aerating agent present at between 1 and 20 dry weight percent.

22. The bio-material composition of claim 21, wherein the aerating agent is present at between 1 and 5 dry weight percent.

23. The bio-material composition of claim 21 or claim 22, wherein the aerating agent is calcium carbonate, sodium carbonate, sodium bicarbonate, calcium bicarbonate, baking soda, baking powder or a combination thereof.

24. The bio-material composition of any one of claims 1-23, further comprising water.

25. The bio-material composition of claim 24, wherein the bio-material is a slurry having a pH between 6 and 8.

26. An orthopedic kit comprising:
the dry bio-material of any one of claims 1-23; and
an aqueous activator solution.

27. A bio-material according to any one of claims 1-25 for use in a method for promoting hard tissue growth comprising the application of said bio-material to a tissue or a hard tissue defect.

28. The biomaterial for use in a method of claim 27, wherein the hard tissue is bone.

## Patentansprüche

1. Osteoproliferative Zusammensetzung aus biologischem Material, umfassend:
eine Phosphorsäure oder ein Phosphorsäuresalz, die bzw. das mit zwischen etwa 20 und 70 Trockengewichtsprozent vorliegt;
ein Metalloxid, das mit zwischen etwa 10 und 50 Trockengewichtsprozent vorliegt;
eine calciumhaltige Verbindung, die mit zwischen etwa 1 und 15 Trockengewichtsprozent vorliegt; und eine zuckerartige Verbindung, die mit zwischen 0,5 und 20 Trockengewichtsprozent vorliegt und aus der aus Zuckern, Zuckeralkoholen, Zuckersäuren, Aminozuckern, Glykosaminoglykaners, Glykolipiden, Zuckerersatzstoffen und Kombinationen davon bestehenden Gruppe ausgewählt ist.

2. Zusammensetzung aus biologischem Material nach Anspruch 1, wobei das Gewichtsverhältnis zwischen der Phosphorsäure oder dem Phosphorsäuresalz und dem Metalloxid zwischen etwa 2:1 und 1:1 beträgt.

3. Zusammensetzung aus biologischem Material nach einem der Ansprüche 1-2, wobei die Phosphorsäure bzw. das Phosphorsäuresalz aus der aus KH₂PO₄, Natriumphosphat, Aluminiumphosphat, Monoammonium-phosphat und Diammoniumphosphat und Kombinationen davon bestehenden Gruppe ausgewählt ist.

4. Zusammensetzung aus biologischem Material nach einem der Ansprüche 1-2, wobei es sich bei der Phosphorsäure bzw. dem Phosphorsäuresalz um KH₂PO₄ handelt.

5. Zusammensetzung aus biologischem Material nach einem der Ansprüche 1-4, wobei das Metalloxid aus der aus MgO, FeO, Al (OH)₃, Fe₂O₃, Fe₃O₄, ZrO, Zr(OH)₄, Zinkoxiden und -hydroxiden, Calciumoxid und -hydroxiden und Kombinationen davon bestehenden Gruppe ausgewählt ist.

6. Zusammensetzung aus biologischem Material nach einem der Ansprüche 1-4, wobei es sich bei dem Metalloxid um MgO handelt.

7. Zusammensetzung aus biologischem Material nach einem der Ansprüche 1-6, wobei die calciumhaltige Verbindung aus der aus : α-Ca₃(PO₄)₂, β-Ca₃(PO₄)₂, Ca₁₀(PO₄)₃(OH)₂, Tetracalciumphosphat, amorphem Calciumphosphat, biphasischem Calciumphosphat, schlecht kristallisiertem Apatit, Oxyapatit, Octacalciumphosphat, Dicaiciumphosphat, Dicalciumphosphat-Dihydrat, Calciummetaphosphat, Heptacalciummetaphosphat, Calciumpyrophosphat und Kombinationen davon bestehenden Gruppe ausgewählt ist.

8. Zusammensetzung aus biologischem Material nach Anspruch 7, wobei es sich bei der calciumhaltigen Verbindung um ein Tricalciumphosphat handelt.

9. Zusammensetzung aus biologischem Material nach Anspruch 8, wobei die calciumhaltige Verbindung aus der aus : α-Ca₃(PO₄)₂, β-Ca₃ (PO₄)₂, Ca₁₀(PO₄)₆(OH)₂ bestehenden Gruppe ausgewählt ist.

10. Zusammensetzung aus biologischem Material nach Anspruch 8, wobei es sich bei dem Tricalciumphosphat um Ca₁₀(PO₄)₆(OH)₂ handelt.

11. Zusammensetzung aus biologischem Material nach einem der Ansprüche 1-2, wobei es sich bei der Phosphorsäure bzw. dem Phosphorsäuresalz um KH₂PO₄;
dem Metalloxid um MgO; und
der calciumhaltigen Verbindung um ein Tricalciumphosphat handelt.

12. Zusammensetzung aus biologischem Material nach einem der Ansprüche 1-11, wobei die zuckerartige Verbindung aus der aus: Zuckern, Zuckeralkoholen, Zuckersäuren, Aminozuckern, Zuckerpolymeren und Kombinationen davon bestehenden Gruppe ausgewählt ist.

13. Zusammensetzung aus biologischem Material nach Anspruch 12, wobei es sich bei der zuckerartigen Verbindung um einen Zucker handelt.

14. Zusammensetzung aus biologischem Material gemäß Anspruch 13, wobei es sich bei der zuckerartigen Verbindung um Saccharose handelt.

15. Zusammensetzung aus biologischem Material nach Anspruch 12, wobei es sich bei der zuckerartigen Verbindung um ein Polysaccharid handelt.

16. Zusammensetzung aus biologischem Material nach einem der Ansprüche 1-15, wobei:
die Phosphorsäure bzw. das Phosphorsäuresalz mit zwischen etwa 40 und 65 Trockengewichtsprozent vorliegt;
das Metalloxid mit zwischen etwa 30 und 50 Trockengewichtsprozent vorliegt;
die calciumhaltige Verbindung mit zwischen etwa 1 und 15 Trockengewichtsprozent vorliegt; und
die zuckerartige Verbindung mit zwischen 0,5 und 20 Trockengewichtsprozent vorliegt.

17. Zusammensetzung aus biologischem Material nach einem der Ansprüche 1-15, wobei:
es sich bei der Phosphorsäure bzw. dem Phosphorsäuresalz um mit zwischen etwa 40 und 50 Trockengewichtsprozent vorliegendes KH₂PO₄ handelt;
das Metalloxid mit zwischen etwa 35 und 50 Trockengewichtsprozent vorliegt;
die calciumhaltige Verbindung mit zwischen etwa 1 und 15 Trockengewichtsprozent vorliegt; und
die zuckerartige Verbindung mit zwischen etwa 0,5 und 10 Trockengewichtsprozent vorliegt.

18. Zusammensetzung aus biologischem Material gemäß einem der Ansprüche 1-17, wobei die zuckerartige Verbindung mit zwischen 0,5 und 10 Trockengewichtsprozent vorliegt.

19. Zusammensetzung aus biologischem Material nach einem der Ansprüche 1-18, wobei die Zusammensetzung osteoinduktiv ist.

20. Zusammensetzung aus biologischem Material nach einem der Ansprüche 1-19, ferner umfassend ein Antibiotikum, einen Wachstumsfaktor oder eine lebende Zelle.

21. Zusammensetzung aus biologischem Material nach einem der Ansprüche 1-20, ferner umfassend ein mit zwischen 1 und 20 Trockengewichtsprozent vorliegendes Belüftungsmittel.

22. Zusammensetzung aus biologischem Material nach Anspruch 21, wobei das Belüftungsmittel mit zwischen 1 und 5 Trockengewichtsprozent vorliegt.

23. Zusammensetzung aus biologischem Material nach Anspruch 21 oder Anspruch 22, wobei es sich bei dem Belüftungsmittel um Calciumcarbonat, Natriumcarbonat, Natriumhydrogencarbonat, Calciumhydrogencarbonat, Natron, Backpulver oder eine Kombination davon handelt.

24. Zusammensetzung aus biologischem Material nach einem der Ansprüche 1-23, ferner umfassend Wasser.

25. Zusammensetzung aus biologischem Material nach Anspruch 24, wobei es sich bei dem biologischen Material um eine Aufschlämmung mit einem pH-Wert zwischen 6 und 8 handelt.

26. Orthopädisches Kit, umfassend:
das trockene biologische Material nach einem der Ansprüche 1-23; und
eine wässrige Aktivatorlösung.

27. Biologisches Material gemäß einem der Ansprüche 1-25 zur Verwendung bei einem Verfahren zur Förderung des Hartgewebewachstums, wobei man das biologische Material auf einen Gewebe- oder einen Hartgewebeschaden anwendet.

28. Biologisches Material zur Verwendung bei einem Verfahren nach Anspruch 27, wobei es sich bei dem Hartgewebe um Knochen handelt.

## Revendications

1. Composition de biomatériaux ostéoproliférative comprenant :
un acide phosphorique ou sel d'acide phosphorique entre environ 20 et 70 pour cent en poids sec ;
un oxyde métallique entre environ 10 et 50 pour cent en poids sec ;
un composé calcique entre environ 1 et 15 pour cent en poids sec ; et
un composé sucré entre 0,5 et 20 pour cent en poids sec et choisi dans le groupe constitué par les sucres, les sucres-alcools, les sucres-acides, les sucres aminés, les glucosaminoglycanes, les glucolipides, les succédanés du sucre et des combinaisons de ceux-ci.

2. Composition de biomatériau selon la revendication 1, **caractérisée en ce que** le rapport en poids entre l'acide phosphorique ou le sel d'acide phosphorique et l'oxyde métallique est compris entre environ 2:1 et 1:1.

3. Composition de biomatériau selon l'une ou l'autre des revendications 1 et 2, **caractérisée en ce que** l'acide phosphorique ou le sel d'acide phosphorique est choisi dans le groupe constitué par le KH₂PO₄, le phosphate de sodium, le phosphate d'aluminium, le phosphate de mono-ammonium et le phosphate de di-ammonium, et des combinaisons de ceux-ci.

4. Composition de biomatériau selon l'une ou l'autre des revendications 1 et 2, **caractérisée en ce que** l'acide phosphorique ou le sel d'acide phosphorique est le KH₂PO₄.

5. Composition de biomatériau selon l'une quelconque des revendications 1 à 4, **caractérisée en ce que** l'oxyde métallique est choisi dans le groupe constitué par le MgO, le FeO, l'Al(OH)₃, le Fe₂O₃, le Fe₃O₄, le ZrO, le Zr(OH)₄, les oxydes et hydroxydes de zinc, l'oxyde et les hydroxydes de calcium et des combinaisons de ceux-ci.

6. Composition de biomatériau selon l'une quelconque des revendications 1 à 4, **caractérisée en ce que** l'oxyde métallique est le MgO.

7. Composition de biomatériau selon l'une quelconque des revendications 1 à 6, **caractérisée en ce que** le composé calcique est choisi dans le groupe constitué par : le α-Ca₃(PO₄)₂, le β-Ca₃(PO₄)₂, le Ca₁₀(PO₄)₃(OH)₂z, le phosphate tétracalcique, le phosphate de calcium amorphe, le phosphate de calcium biphasique, l'apatite mal cristallisée, l'oxyapatite, le phosphate octacalcique, le phosphate dicalcique, le phosphate dicalcique dihydraté, le métaphosphate de calcium, le métaphosphate héptacalcique, le pyrophosphate de calcium et des combinaisons de ceux-ci.

8. Composition de biomatériau selon la revendication 7, **caractérisée en ce que** le composé calcique est le phosphate tricalcique.

9. Composition de biomatériau selon la revendication 8, **caractérisée en ce que** le composé calcique est choisi dans le groupe constitué par : le α-Ca₃(PO₄)₂, le β-Ca₃(PO₄)₂, le Ca₁₀(PO₄)₆(OH)₂.

10. Composition de biomatériau selon la revendication 8, **caractérisée en ce que** le phosphate tricalcique est le Ca₁₀(PO₄)₆(OH)₂.

11. Composition de biomatériau selon l'une ou l'autre des revendications 1 et 2, **caractérisée en ce que** l'acide phosphorique ou le sel d'acide phosphorique est le KH₂PO₄ ;
l'oxyde métallique est le MgO ; et
le composé calcique est un phosphate tricalcique.

12. Composition de biomatériau selon l'une quelconque des revendications 1 à 11, **caractérisée en ce que** le composé sucré est choisi dans le groupe constitué par : les sucres, les sucres-alcools, les sucres-acides, les sucres aminés, les sucres - polymères, et des combinaisons de ceux-ci.

13. Composition de biomatériau selon la revendication 12, **caractérisée en ce que** le composé sucré est un sucre.

14. Composition de biomatériau selon la revendication 13, **caractérisée en ce que** le composé sucré est le saccharose.

15. Composition de biomatériau selon la revendication 12, **caractérisée en ce que** le composé sucré est un polysaccharide.

16. Composition de biomatériau selon l'une quelconque des revendications 1 à 15, **caractérisée en ce que** :
l'acide phosphorique ou le sel d'acide phosphorique est entre environ 40 et 65 pour cent en poids sec ;
l'oxyde métallique est entre environ 30 et 50 pour cent en poids sec ;
le composé calcique est entre environ 1 et 15 pour cent en poids sec ; et
le composé sucré est entre 0,5 et 20 pour cent en poids sec.

17. Composition de biomatériau selon l'une quelconque des revendications 1 à 15, **caractérisée en ce que** :
l'acide phosphorique ou le sel d'acide phosphorique est KH₂PO₄ entre environ 40 et 50 pour cent en poids sec ;
l'oxyde métallique est entre environ 35 et 50 pour cent en poids sec ;
le composé calcique est entre environ 1 et 15 pour cent en poids sec ; et
le composé sucré est entre 0,5 et 10 pour cent en poids sec.

18. Composition de biomatériau selon l'une quelconque des revendications 1 à 17, **caractérisée en ce que** le composé sucré est entre 0,5 et 10 pour cent en poids sec.

19. Composition de biomatériau selon l'une quelconque des revendications 1 à 18, **caractérisée en ce que** la composition est ostéoinductrice.

20. Composition de biomatériau selon l'une quelconque des revendications 1 à 19, comprenant en outre un antibiotique, un facteur de croissance, ou une cellule vivante.

21. Composition de biomatériau selon l'une quelconque des revendications 1 à 20, comprenant en outre un agent d' aération entre 1 et 20 pour cent en poids sec.

22. Composition de biomatériau selon la revendication 21, **caractérisée en ce que** l'agent d'aération est entre 1 et 5 pour cent en poids sec.

23. Composition de biomatériau selon la revendication 21 ou la revendication 22, **caractérisée en ce que** l'agent d'aération est le carbonate de calcium, le carbonate de sodium, le bicarbonate de sodium, le bicarbonate de calcium, le bicarbonate de soude, la poudre à lever, ou une combinaison de ceux-ci.

24. Composition de biomatériau selon l'une quelconque des revendications 1 à 23, comprenant en outre de l'eau.

25. Composition de biomatériau selon la revendication 24, **caractérisée en ce que** le biomatériau est une bouillie ayant un pH compris entre 6 et 8.

26. Kit orthopédique comprenant :
le biomatériau sec selon l'une quelconque des revendications 1 à 23 ; et
une solution aqueuse d'activateur.

27. Biomatériau selon l'une quelconque des revendication 1 à 25, destiné à être utilisé dans une méthode pour favoriser la croissance de tissus durs comprenant l'application dudit biomatériau à un tissu ou un défaut du tissu dur.

28. Biomatériau destiné à être utilisé dans une méthode selon la revendication 27, **caractérisé en ce que** le tissu dur est l'os.
